Europäisches Patentamt

European Patent Office

Office européen des brevets

(11) Publication number: **0 292 291 B1**

# EUROPEAN PATENT SPECIFICATION

(45) Date of publication of patent specification: **10.08.94**

(21) Application number: **88304551.0**

(22) Date of filing: **19.05.88**

(51) Int. Cl.⁵: **C07K 7/00**, C07K 7/24,
//C07K99/10, A61K37/02,
A61K7/00

(54) **Linear and cyclic analogs of alpha-msh fragments with extraordinary potency.**

(30) Priority: **22.05.87 US 53229**

(43) Date of publication of application:
**23.11.88 Bulletin 88/47**

(45) Publication of the grant of the patent:
**10.08.94 Bulletin 94/32**

(84) Designated Contracting States:
**AT BE CH DE ES FR GB GR IT LI LU NL SE**

(56) References cited:
**US-A- 4 485 039**

**CHEMICAL ABSTRACTS, vol. 101, no. 21, 19th
November 1984, page 90, abstract no.
184199e, Columbus, Ohio, US; B.E.B. SAND-
BERG et al.: "A conformational approach to
structure-activity studies of substance P", &
SUBST. P--DUBLIN 1983, PROC. INT. SYMP.
1983, 18-19**

(73) Proprietor: **UNIVERSITY PATENTS, INC.
1465 Post Road East,
P.O. Box 901
Westport, Connecticut 06881 (US)**

(72) Inventor: **Hruby, Victor J.
2802 E. Via Rotunda
Tucson Arizona (US)**
Inventor: **Al-Obeidi, Fahad A.
770 North Dodge
Tucson Arizona (US)**
Inventor: **Hadley, Mac E.
1921 Calle Campana de Plata
Tucson Arizona (US)**

(74) Representative: **Allam, Peter Clerk et al
LLOYD WISE, TREGEAR & CO.
Norman House
105-109 Strand
London WC2R 0AE (GB)**

Rank Xerox (UK) Business Services
(3.10/3.09/3.3.3)

CHEMICAL ABSTRACTS, vol. 105, no. 11, 15th September 1986, page 89, abstract no. 91517z, Columbus, Ohio, US; D.G. KLEMES et al.: "Potent and prolonged melanotropic activities of the alpha-MSH fragment analog, Ac-[Nlc4, beta-Phe7]-alpha-MSH,-NH2", & BIOCHEM. BIOPHYS. RES. COMMUN. 1986, 137(2), 722-8

CHEMICAL ABSTRACTS, vol. 97, no. 3, 19th July 1982, page 102, abstract no. 17366z, Columbus, Ohio, US; T.K. SAWYER et al.: "Conformation-activity studies of alpha-melanocyte stimulating hormone: synthesis of a cyclic alpha-melanotropin exhibiting high potency and full biological activity", & PEPT.: SYNTH., STRUCT., FUNCT., PROC. AM. PEPT. SYMP. 7th 1981, 387-90

CHEMICAL ABSTRACTS, vol. 100, no. 19, 7th May 1984, pages 58-59, abstract no. 151154n, Columbus, Ohio, US; B.C. WILKES et al.: "Differentiation of the structural features of melanotropins important for biological potency and prolonged activity in vitro", & INT. J. PEPT. PROTEIN RES. 1983, 22(3), 313-24

**Description**

In view of the partial support provided by grants from the United States Public Health Service and the National Science Foundation in the making of the present invention, the United states Government has certain rights to this technology under 35 USC 202.

The present invention concerns a new class of previously unreported linear and cyclic fragment analogues of alpha-MSH, the method of stimulating melanocytes in vertebrates by the transdermal application of these analogues, and compositions useful in the method.

In vertebrates, the color of their skin, fur, and feathers is determined by the number and distribution of certain color-bearing cells, e.g. melanocytes, the number and distribution of which is under genetic control. Melanocytes in mammals are localized at the basal layer of the epidermis, at the dermal-epidermal junction, and within hair follicles. Synthesis of pigment (melanin) within these melanocytes is controlled by the activity of an enzyme, tyrosinase, which is localized in an intracellular organelle, the premelanosome. Upon activation of tyrosinase, either eumelanin (brown-black) or phaeomelanin (yellow-red) pigment is deposited within the organelle; after complete melanization, the premelanosome is known as a melanosome, more specifically either an eumelanosome or a phaeomelanosome depending upon color [see Fitzpatrick, T. B., Y. Hori, K. Toda, M. Seiji, Jap. J. Derm. 79:278(1969)]. Melanosomes are delivered to surrounding keratinocytes of the skin or to cells within the shaft of the growing hair by the process known as cytocrine secretion.

Although melanin synthesis and pelage patterns are expressed genetically, follicular melanogenesis and pelage color changes in some mammals may be hormonally controlled by alpha-melanotropin (also known as alpha-melanocyte stimulating hormone, i.e. alpha-MSH), a linear tridecapeptide of the formula:

$$\text{Ac-Ser-Tyr-Ser-Met-Glu-His-Phe-Arg-Trp-Gly-Lys-Pro-Val-NH}_2.$$

This hormone is derived from a large molecular weight precursor protein, proopiomelanocortin, and is secreted by the pars intermedia of the pituitary gland, and stimulates melanocyte adenylate cyclase activity, tyrosinase activity, and subsequent melanin production [see Hadley, M. E., C. B. Heward, V. J. Hruby, T. K. Sawyer, and Y. C. S. Young, Pigment Cell 6:323(1980)].

In humans, alpha-MSH is apparently found only in the pituitary gland of the fetus and not in the adult pituitary gland. In adult humans, a certain level of melanin production is genetically determined and constitutively present. Variable melanin synthesis above and beyond this baseline level is directly dependent on UV stimulation, e.g. sunlight; exposure to high levels of sun triggers increased production of melanin, with concomittant darkening of the skin. This response may be an evolutionary adaptation to protect the person against the aging and mutagenic properties of UV. Exposure to low levels of UV results in lower levels of integumental melanin synthesis, fading of skin color, and a diminished blocking effect allowing the skin to absorb greater amounts of radiation. Although adults do not synthesize alpha-MSH in the pituitary gland, human melanocytes will respond to this hormone (and a racemized preparation thereof).

Hypopigmentation of the skin in humans results from local defects in melanin production within the melanocytes; however, the etiology for many such hypopigmentary disturbances is still unknown.

It is estimated that approximately 1% of the world's population is afflicted with some form of hypopigmentation dysfunctions. Although it is known that alpha-MSH and certain analogues of alpha-MSH can cause darkening in amphibians when administered subcutaneously, and that alpha-MSH is associated with skin darkening in adrenalectomized humans when administered intramuscularly [Lerner, A. B., and J. S. McGuire, N. E. J. Med. 270:539-546(1964)], these routes of administration are not suitable for repeated application necessary to achieve and maintain the desired effect. Prior to the present invention no adequate means of treating these hypopigmentation disorders were known.

It has now been discovered that certain analogues of alpha-MSH can effectively be administered transcutaneously, and these compounds will reach the melanocytes in active form to stimulate the production of melanin. Thus, according to the present invention, it is now possible and convenient to apply topical compositions comprising alpha-MSH analogues to achieve normalization of hypopigmentation dysfunctions such as postinflammatory hypopigmentation, including pityriasis, alba, tinea versiocolor, vitiligo, idiopathic guttate hypomelanosis; and nevus depigmentosus. Furthermore, it is now possible to achieve darkening of grey hair due to aging by topical application of alpha-MSH analogues. It is also possible to enhance the value of commercial animal pelts by darkening via topical application of these analogues. In addition, it is now possible to achieve darkening of the skin in the total absence of sun or UV light irradiation.

All previous studies of the peptides related to alpha-MSH isolated from the pituitary gland showed the conservation of the $Met^4$-$Glu^5$-$His^6$-$Phe^7$-$Arg^8$-$Trp^9$-$Gly^{10}$ sequence (that is Methionine-Glutamic acid-Histidine-Phenylalanine-Arginine-Tryptophan-Glycine) as the common active core. This heptapeptide sequence has been suggested to be the active site of the hormones derived from proopiomelanocortin.

Based upon the structural relationship of alpha-MSH (and its analogues and fragments) to its biological potency, using in vitro a frog skin bioassay system, the active site of alpha-MSH appears to be the common active core sequence suitably modified Ac-Met-Glu-His-Phe-Arg-Trp-Gly-$NH_2$. This fragment (Alpha-$MSH_{4-10}$) has been synthesized and tested for its melanocyte stimulating activity in frog skin and shown to be a weak agonist when compared to the native hormone. The amino acid exchanges in position 5(Glu) and 10(Gly) have not, previous to the making of the present invention, been investigated. From previous research (see United States Patents Nos. 4,457,864, 4,485,039, 4,866,038, 4,918,055 and 5,049,547) it was found that the replacement of methionine with norleucine in the active linear core heptapeptide gave a potent analogue of alpha-MSH. In addition, substitution of phenylanine at position 7 with its enantiomer D-phenylalanine, resulted in a more biologically potent analogue having prolonged activity in both recognized (frog and lizard skin) bioassays. The following table summarizes some of these studies.

| Selective Fragments of Alpha-MSH and Their Biological Activities on Frog (Rana pipiens) Skin and Lizard (Anolis carolinensis) Skin | | |
|---|---|---|
| Peptide | Relative Potency | |
| | Frog | Lizard |
| alpha-MSH | 1.0 | 1.0 |
| Ac-[$Nle^4$,D-$Phe^7$]-alpha-$MSH_{1-13}NH_2$ | 60.0 | 5.0 |
| Ac-alpha-$MSH_{4-10}NH_2$ | 0.0003 | 0.004 |
| Ac-[$Nle^4$]-alpha-$MSH_{4-10}NH_2$ | 0.002 | 0.06 |
| Ac-[$Nle^4$]-alpha-$MSH_{4-11}NH_2$ | 0.002 | 1.0 |
| Ac-[$Nle^4$,D-$Phe^7$]-alpha-$MSH_{4-10}NH_2$ | 0.02 | 10.0 |
| Ac-[$Nle^4$,D-$Phe^7$]-alpha-$MSH_{4-11}NH_2$ | 0.16 | 8.0 |

The investigation of these structural-activity relationships showed two important factors: (1) the replacement of the Met amino acid, with its oxidizable side chain, with the oxidatively stable amino acid Nle, which is also an isostere for Met, resulted in about 10 times enhancement of the biological activity of the peptide fragment; and (2) replacement of L-Phe in position 7 with D-Phe and keeping Lys in position 11 further enhances the biological activity of alpha-MSH.

In addition to the studies conducted on linear alpha-MSH analogues, a number of conformationally constrained alpha-MSH compounds have been synthesized and tested for biological potency. The first analogue studied, was

$$Ac-[\overline{Cys^4,Cys^{10}}]-alpha-MSH_{1-13}NH_2$$

which had about 10-20 times the activity of the native alpha-MSH hormone in the frog skin bioassay and about two-fold the potency of alpha-MSH in the lizard skin bioassay. The design and synthesis of these cyclic alpha-MSH analogues were based on the consideration of the beta-turn structure at the center of the active core ($His^6$-$Phe^7$-$Arg^8$-$Trp^9$) of alpha-MSH, and the importance of this conformation pattern for biological activity.

4

From structure activity studies of this cyclic class of alpha-MSH analogues, several conclusions ware drawn: 1) cyclization between positions 4 (Met) and 10 (Gly) by isosteric replacement with cysteine amino acid enhanced the melanocyte dispersion activity by not less than 10 times in frog skin bioassay and two times in lizard skin; 2) substitution of Phe[7] with D-Phe[7] in these cyclic analogues doubles the activity in the frog skin bioassay and increases it four times in the lizard skin bioassay; 3) the presence of Lys in position 11 always gave more active analogues than those without it; and 4) reduction or expansion of the ring size of the disulfide bridge from a 23-membered ring causes severe reduction in the biological potency of the resultant analogue.

The results of these studies with cyclic alpha-MSH analogues are given in the following table:

Relative in Vitro Potencies of Cyclic Alpha-MSH
Analogues in the Frog and Lizard Skin Bioassays

| Peptide Analogue | Potency* | |
| --- | --- | --- |
| | Frog | Lizard |
| Amino Acids Substitution Effect | | |
| alpha-MSH | 1.0 | 1.0 |
| Ac-[Cys$^4$, Cys$^{10}$]-alpha-MSH$_{1-13}$NH$_2$ | 10.0 | 2.0 |
| Ac-[Cys$^4$, Cys$^{10}$]-alpha-MSH$_{4-13}$NH$_2$ | 30.0 | 0.6 |
| Ac-[Cys$^4$, D-Phe$^7$, Cys$^{10}$]-alpha-MSH$_{4-13}$NH$_2$ | 6.0 | 6.0 |
| Ac-[Cys$^4$, Cys$^{10}$]-alpha-MSH$_{4-12}$NH$_2$ | 10.0 | 1.5 |
| Ac-[Cys$^4$, D-Phe$^7$, Cys$^{10}$]-alpha-MSH$_{4-12}$NH$_2$ | 20.0 | 6.0 |
| Ac-[Cys$^4$, Cys$^{10}$]-alpha-MSH$_{4-11}$NH$_2$ | 0.16 | 0.07 |
| Ac-[Cys$^4$, D-Phe$^7$, Cys$^{10}$]-alpha-MSH$_{4-11}$NH$_2$ | 2.5 | 3.0 |
| Ac-[Cys$^4$, Cys$^{10}$]-alpha-MSH$_{4-10}$NH$_2$ | 0.06 | 0.003 |
| Ac-[Cys$^4$, D-Phe$^7$, Cys$^{10}$]-alpha-MSH$_{4-10}$NH$_2$ | 0.75 | 0.5 |

Ac-[Cys$^4$, Cys$^{10}$]-alpha-MSH$_{4-13}$NH$_2$      30.0      0.60

[Mpa$^4$, Cys$^{10}$]-alpha MSH$_{4-13}$NH$_2$      30.0      1.0

[Maa$^4$, Cys$^{10}$]-alpha-MSH$_{4-13}$NH$_2$      0.06      0.06

Ac-[Hcy$^4$, Cys ]-alpha-MSH$_{4-13}$NH$_2$      0.06      0.70

* Biological potencies are measured relative to alpha-MSH over the linear portion of the dose-response curve.

    Maa = 2-Mercaptoacetic acid

    Mpa = 3-Mercaptopropionic acid

    Hcy = Homocysteine

To further investigate the structure-potency relationship of alpha-MSH$_{4-10}$ fragments, we have systematically investigated the structural requirement for melanotropic activity of a number of previously unreported linear alpha-MSH$_{4-10}$ and alpha-MSH$_{4-13}$ analogues with emphasis on the effect of substitution at positions 5 and 10.

Based on the results obtained for linear alpha-MSH analogues, we next investigated a different kind of conformationally restricted alpha-MSH analogue. Briefly, we made and concentrated on 4-10 fragments of alpha-MSH with the general structure:

    Ac[Nle$^4$, Xxx$^5$, D-Phe$^7$, Yyy$^{10}$]alpha-MSH$_{4-10}$NH$_2$

where X$_{xx}$ is either glutamic acid (Glu) or aspartic acid (Asp) (that is one of the mono-amino dicarboxylic acids), and Y$_{yy}$ is lysine, ornithine, 2,4-diaminobutyric acid (Dab), or 2,3-diaminopropionic acid (Dpr), i.e. a dibasic amino acid. The alpha-MSH linear analogues listed in the following tables were synthesized by solid-phase peptide synthetic methods using p-methylbenzhydrylamine resin as a solid support, and purified by methods related to those used previously for alpha-melanotropin and analogues. For comparison the structure of alpha-MSH is given first.

EP 0 292 291 B1

## Structure of Alpha-Melanotropin Analogues

| No. | Primary Sequence |
|-----|------------------|

| | 1 | 2 | 3 | 4 | 5 | 6 | 7 | 8 | 9 | 10 | 11 | 12 | 13 |
|---|---|---|---|---|---|---|---|---|---|----|----|----|----|

alpha-MSH    Ac-Ser-Tyr-Ser-Met-Glu-His-Phe-Arg-Trp-Gly-Lys-Pro-Val-NH$_2$

1    Ac-Ser-Tyr-Ser-Nle-Glu-His-D-Phe-Arg-Trp-Lys-Gly-Pro-Val-NH$_2$

2    Ac-Ser-Tyr-Ser-Nle-Asp-His-D-Phe-Arg-Trp-Lys-Gly-Pro-Val-NH$_2$

3    Ac-Nle-Glu-His-D-Phe-Arg-Trp-Lys-Gly-Pro-Val-NH$_2$

4    Ac-Nle-Asp-His-D-Phe-Arg-Trp-Lys-Gly-Pro-Val-NH$_2$

5    Ac-Nle-Asp-His-D-Phe-Arg-Trp-Gly-NH$_2$

6    Ac-Nle-Glu-His-D-Phe-Arg-Trp-Lys-NH$_2$

7    Ac-Nle-Asp-His-D-Phe-Arg-Trp-Lys-NH$_2$

8    Ac-Nle-Glu-His-D-Phe-Arg-Trp-Orn-NH2

9    Ac-Nle-Asp-His-D-Phe-Arg-Trp-Orn-NH$_2$

10    Ac-Nle-Glu-His-D-Phe-Arg-Trp-Dab-NH$_2$

11    Ac-Nle-Asp-His-D-Phe-Arg-Trp-Dab-NH$_2$

12    Ac-Nle-Asp-His-D-Phe-Arg-Trp-Dpr-NH$_2$

13    Ac-Nle-Glu-His-Phe-Arg-Trp-Lys-NH$_2$

14    Ac-Nle-Asp-His-Phe-Arg-Trp-Lys-NH$_2$

| Analogue |
| --- |
| alpha-MSH |
| 1. Ac-[Nle$^4$, D-Phe$^7$, Lys$^{10}$, Gly$^{11}$]-alpha MSH$_{1-13}$NH$_2$ |
| 2. Ac-[Nle$^4$, Asp$^5$, D-Phe$^7$, Lys$^{10}$, Gly$^{11}$]-alpha-MSH$_{1-13}$NH$_2$ |
| 3. Ac-[Nle$^4$, D-Phe$^7$, Lys$^{10}$, Gly$^{11}$]-alpha-MSH$_{4-13}$NH$_2$ |
| 4. Ac-[Nle$^4$, Asp$^5$, D-Phe$^7$, Lys$^{10}$, Gly$^{11}$]-alpha-MSH$_{4-13}$NH$_2$ |
| 5. Ac-[Nle$^4$, Asp$^5$, D-Phe$^7$]-alpha-MSH$_{4-10}$NH$_2$ |
| 6. Ac-[Nle$^4$, D-Phe$^7$, Lys$^{10}$]-alpha-MSH$_{4-10}$NH$_2$ |
| 7. Ac-[Nle$^4$, Asp$^5$, D-Phe$^7$, Lys$^{10}$]-alpha-MSH$_{4-10}$NH$_2$ |
| 8. Ac-[Nle$^4$, D-Phe$^7$, Orn$^{10}$]-alpha-MSH$_{4-10}$NH$_2$ |
| 9. Ac-[Nle$^4$, Asp$^5$, D-Phe$^7$, Orn$^{10}$]-alpha-MSH$_{4-10}$NH$_2$ |
| 10. Ac-[Nle$^4$, D-Phe$^7$, Dab$^{10}$]-alpha-MSH$_{4-10}$NH$_2$ |
| 11. Ac-[Nle$^4$, Asp$^5$, D-Phe$^7$, Dab$^{10}$]-alpha-MSH$_{4-10}$NH$_2$ |
| 12. Ac-[Nle$^4$, Asp$^5$, D-Phe$^7$, Dpr$^{10}$]-alpha-MSH$_{4-10}$NH$_2$ |
| 13. Ac-[Nle$^4$, Lys$^{10}$]-alpha-MSH$_{4-10}$NH$_2$ |
| 14. Ac-[Nle$^4$, Asp$^5$, Lys$^{10}$]-alpha-MSH$_{4-10}$NH$_2$ |

The linear analogues of alpha-MSH fragments were manufactured according to the following example:

EXAMPLE I

Ac-[Nle$^4$, D-Phe$^7$, Lys$^{10}$, Gly$^{11}$]-alpha-MSH$_{1-13}$NH$_2$

This compound was prepared by coupling N$^\alpha$-Boc-Val (the term "Boc" means t-butyloxycarbonyl) to p-methylbenzhydrylamine resin (2.0 g pMBHA resin, 0.7 mmol NH$_2$/g of resin) using a 3 fold excess of amino acid and using solid-phase methods of peptide synthesis. After 90 min the resin washed with dichloromethane, neutralized and the amino group acetylated with acetic anhydride-pyridine mixture. No reactive amino groups on the resin were detected by the ninhydrin test after 30 min. A cycle for coupling of each amino acid residue into the growing peptide chain consisted of the following: 1) Washing with four 30-mL portions of $CH_2Cl_2$, 2 min/wash; 2) Cleavage of the Boc group by 30 ml of 48% trifluoroacetic acid in dichloromethane containing 2% anisole, one treatment for 5 min, a second for 20 min; 3) Washing with four 30-mL portions of dichloromethane, 2 min/wash; 4) neutralization by addition of two 30-mL portions of 10% diisopropylethylamine in dichloromethane and shaking for 2 min each; 5) Washing with four 30-mL portions of dichloromethane, 2 min/wash; 6) Addition of 3 folds of the Boc amino derivative (in this case 2.1 mmol) in 5 ml of dichloromethane, 2.4 ml of N-hydroxybenzotriazole of 1 mmol/mL solution of HOBt in DMF (except in the case of N-Boc-$N^{im}$Tos His), (the term "$N^{im}$Tos" means N-imidazole tosyl), followed by 2.4 ml of DCC of 1 mmol/ml solution of DCC in DMF. The mixture then shook for 2-3 h (in case of Trp, Arg, and His, DMF was used as a coupling solvent); 7) After completion of the coupling (ninhydrin negative) washing with three 30-mL portions of dichloromethane, 2 min/wash; 8), washing with 3 ml portion of 100% ethanol, 2 min/wash; 9) Washing with four 30-mL portions of dichloromethane, 1 min/wash. The protected peptide resin corresponding to the title compound was obtained after stepwise coupling of the following $N^{\alpha}$-Boc amino acids (or derivatives) was performed (in order of addition): $N^{\alpha}$-Boc-Pro; $N^{\alpha}$-Boc-Gly, $N^{\alpha}$-Boc-Lys-($N^{\epsilon}$-ClZ); $N^{\alpha}$-Boc-$N^i$-For-Trp, $N^{\alpha}$-Boc-$N^g$-Tos-Arg, $N^{\alpha}$-Boc-D-Phe, $N^{\alpha}$-Boc-$N^{im}$-Tos-His (the term "$N^g$" means N-guanidino; "$N^i$" means N-indolyl, and "2ClZ" means 2 - chlorobenzyloxycarbonyl). The resulting Boc-His ($N^{im}$-Tos)-D-Phe-Arg ($N^g$-Tos)-Trp ($N^i$-For)-Lys ($N^{\epsilon}$-2ClZ)-Gly-Pro-Val-p-MBHA resin was divided into four portions. One-quarter (1.0g ~ 0.5 mmol) of the protected peptide-resin was converted to the protected title peptide resin after coupling $N^{\alpha}$-Boc-$\gamma$-Bzl-Glu, $N^{\alpha}$-Boc-Nle, $N^{\alpha}$-Boc-Ser (0-Bzl), $N^{\alpha}$-Boc-Tyr (0-2 BrZ); $N^{\alpha}$-Boc-Ser(0-Bzl)(the term "2BrZ" means 2-bromobenzyloxycarbonyl). After coupling the last amino acid, the $N^{\alpha}$-Boc protecting group was removed, the amino group neutralized, and the protected peptide was $N^{\alpha}$-acetylated with a 10-fold excess of N-acetylimidazole in 20 ml of dichloromethane and the resulting protected peptide resin, Ac-Ser(0-Bzl)-Tyr(0-2-BrZ)-Ser(0-Bzl)-Nle-Glu ($\gamma$-Bzl)-His($N^{im}$-Tos)-D-Phe-Arg($N^g$-Tos)-Trp($N^i$-For)-Lys ($N^{\epsilon}$-2-ClZ)-Gly-Pro-Val-p-MBHA resin, dried in vacuo. The protected peptide resin (1.0 g) was cleaved from the resin by liquid HF. After evaporation of the volatile materials in vacuo at 0°C, the dried product was washed with ethyl ether (3 x 30 ml), extracted with 30% aqueous HOAc (3 x 30 ml), and lyophilized. The peptide powder (530 mg) was divided into two portions (260 mg each) and one portion was dissolved in 1.5 ml of ammonium acetate buffer (pH 4.5), filtered through a cartridge filter into the top of a CMC column (2.0 x 30.0 cm) and eluted using 250 ml each of 0.01 M (pH 4.5), 0.1 M (pH 6.8), and 0.2 M (pH 6.8) $NH_4OAc$. The major peak detected at 280 nm, was eluted between the end of the 0.1 and the first half of the 0.2 M $NH_4OAc$ fraction and was lyophilized to give 142.3 mg of white powder. 80.0 mg of the peptide powder was purified by preparative HPLC and the major peak collected and lyophilized to give 63 mg of the title peptide.

EXAMPLE II

Ac-[Nle⁴, Asp⁵, D-Phe⁷, Lys¹⁰, Gly¹¹]-alpha-MSH$_{1-13}$NH$_2$

This compound was prepared from 1.0g (~0.5 mmol) of $N^{\alpha}$-Boc-His($N^{im}$-Tos)-D-Phe-Arg($N^g$-Tos)-(Trp-$N^i$-For)-Lys($N^{\epsilon}$-2-ClZ)-Gly-Pro-Val-p-MBHA resin by stepwise coupling of $N^{\alpha}$-Boc-Asp(0-Bzl), $N^{\alpha}$-Boc-Nle, $N^{\alpha}$-Boc-Ser(0-BZl), $N^{\alpha}$-Boc-Tyr(0-2-BrZ), $N^{\alpha}$Boc-Ser(0-Bzl). Each coupling was achieved by the same approach mentioned previously. Acetylation of the protected tridecapapeptide-resin was carried out by 10-fold excess of N-acetylimidazole in dichloromethane (5 h) after deprotection and neutralization of N-terminal Boc group. The Ac-Ser(0-Bzl)-Tyr(0-2-BrZ)-Ser(0-Bzl)-Nle-Asp(0-Bzl)-His($N^{im}$-Tos)-D-Phe-Arg($N^g$-Tos)-Trp (N$^i$-For)-Lys($N^{\epsilon}$-2-ClZ)-Gly-Pro-Val-p-MBHA resin was dried in vacuo to give 1.8 g of protected peptide resin. A 1.0 g of the protected peptide resin was cleaved by liquid HF and upon evaporation of the volatile materials, washed with diethylether (3 x 30 ml), the peptide extracted of with 30% aqueous HOAc (3 x 30 ml), and lyophilized. A portion of the crude tridecapeptide (200 mg) was dissolved in 1.5 ml, of $NH_4OAc$ buffer (pH 4.5), filtered through a cartridge filter into the top of the CMC column (2.0 x 30.0 cm) and eluted with the same sequence of the discontinuous gradient of $NH_4OAc$ as mentioned in Example I. The separated peptide after lyophilization was 152 mg. A 100 mg of this crude peptide was further purified by HPLC to give 67 mg of the title peptide.

9

EXAMPLE III

Ac-[Nle$^4$, D-Phe$^7$, Lys$^{10}$, Gly$^{11}$]-alpha-MSH$_{4-13}$NH$_2$

From 1.0 g ( 0.5 mmol) of Boc-His(N$^{im}$-Tos)-D̲-Phe-Arg(N$^g$-Tos)-Trp(N$^i$-For)-Lys(N$^\epsilon$-2-ClZ)-Gly-Pro-Val-p-MBHA resin the title peptide was synthesized by solid-phase technique after stepwise coupling of N$^\alpha$-Boc-Glu($\gamma$-Bzl), and N$^\alpha$-Boc-Nle. Each coupling reaction was achieved in the previous examples except that the acetylation of the N-terminous was achieved by 2-fold excess of 1:1 acetic anhydride-pyridine in dichloromethane for 1 h. The peptide 4 was obtained in purified form as outlined in Example II to give a white powder with a yield of 22%.

EXAMPLE IV

Ac-[Nle$^4$, Asp$^5$, D-Phe$^7$, Lys$^{10}$, Gly$^{11}$]-alpha-MSH$_{4-13}$NH$_2$

From 1.0 g (~0.5 mmol) of Boc-His(N$^{im}$-Tos)-D̲-Phe-Arg(N$^g$-Tos)-Trp(N$^i$-For)-Lys(N$^\epsilon$-2-ClZ)-Gly-Pro-Val-p-MBHA resin prepared as in Example I, the title peptide was prepared by stepwise coupling of N$^\alpha$-Boc-Asp($\beta$-Bzl), and N$^\alpha$-Boc-Nle. The same technique was used as described in Example I in processing the resulting protective peptide resin. The desired peptide yielded 53 mg of HPLC pure peptide starting with 130 mg of crude peptide purified previously with CMC chromotography as outlined in Example I.

EXAMPLE V

Ac-[Nle$^4$, D-Phe$^7$, Lys$^{10}$]-alpha-MSH$_{4-10}$NH$_2$

A 2.7 g resin of p-methylbenzhydrylamine (0.7 mmol NH$_2$/g resin) was suspended in dichloromethane and washed three times with 30 ml portions of dichloromethane. The washed resin was then shaken for 2 h in dichloromethane before filtering out the solvent. The swelled MBHA resin was neutralized and coupled with the amino acids as outlined in Example I. The following amino acid derivatives were coupled into the resin (in order of their coupling): N$^\alpha$-Boc-Lys(N$^\epsilon$-2-ClZ), N$^\alpha$-Boc-Trp(N$^i$-For), N$^\alpha$-Boc-Arg(N$^g$-Tos), N$^\alpha$-Boc-D-Phe, N$^\alpha$-Boc-His(N$^{im}$-Tos). The resulting Boc-His(N$^{im}$-Tos)-D-Phe-Arg(N$^g$-Tos)-Trp(N$^i$-For)-Lys(N$^\epsilon$-2-ClZ)-p-MBHA resin was divided into two portions. One part of the resin was coupled stepwise: N$^\alpha$-Boc-Glu($\gamma$-Bzl) and N$^\alpha$-Boc-Nle. The finished peptide resin was debocylated and the N-terminal acetylated with 2-fold excess of 1:1 mixture of acetic anhydride-pyridine in dichloromethane for 1 h. The finished protected peptide resin was washed with dichloromethane and dried in̲ vacuo̲ to give 2.1 g. A 1.7 g of the protected peptide resin was cleaved by anhydrous liquid HF-anisole-dithioethane (17 ml HF, 2 ml anisole, 1 ml dithioethane). After evaporation of the volatile materials, the dried, cleaved peptide was washed with 3 x 30 ml of anhydrous diethylether and extracted with 3 x 30 ml of 30% aqueous HOAc. The aqueous extract of the peptide lyophilized to give 700 mg of crude peptide. A 300 mg sample of the crude peptide was dissolved in 2 ml of NH$_4$OAc buffer (pH 4.5), and filtered through a cartridge filter on the top of CMC column. The major peak was collected and lyophilized to give 172 mg of white powder peptide. 110 mg of the crude peptide was purified on HPLC to give 50 mg of pure title peptide.

EXAMPLE VI

Ac-[Nle$^4$ Asp$^5$, D-Phe, Lys$^{10}$]-alpha-MSH$_{4-10}$NH$_2$

The protected peptide resin to the title compound was prepared from 1.8 g of Boc-His(N$^{im}$-Tos)-D̲-Phe-Arg-(N$^g$-Tos)-Trp(N$^i$-For)-Lys(N$^\epsilon$-2-ClZ)-p-MBHA by stepwise coupling of N$^\alpha$-Boc-Asp($\beta$-Bzl), and N$^\alpha$-Boc-Nle. Each coupling reaction was achieved with a 3-fold excess of N$^\alpha$-Boc amino acid (or derivative), a 2.4-fold excess of DCC, and a 2.4 fold excess of HOBt following the same strategy outlined previously. After coupling the last amino acid, the N$^\alpha$-Boc protection group was removed, the amino group neutralized, and acetylated with 2-fold excess of 1:1 mixture of acetic anhydride pyridine in dichloromethane for 1 h. The Ac-Nle-Asp($\beta$-Bzl)-His(N$^{im}$-Tos)-D̲-Phe-Arg(N$^g$-Tos)-Trp-(N$^i$-For)-Lys(N$^\epsilon$-2-ClZ)-p-MBHA resin was washed with dichloromethane and dried in̲ vacuo̲ to give 2.1 g. A 1.5 g sample of the protected peptide resin was cleaved by liquid HF and processed as in Example V to give 64 mg of the title peptide after HPLC purification of 112 mg of CMC chromatographically pure peptide.

EXAMPLE VII

Ac-[Nle$^4$, Asp$^5$, D-Phe$^7$, Dab$^{10}$]-alpha-MSH$_{4-10}$NH$_2$

The protected peptide resin to the title compound was synthesized as Example V except the N$^\alpha$-Boc-Dab(N$_\gamma$ -Z) and N$^\alpha$-Boc-Asp($\beta$-Bzl) were used instead of N$^\alpha$ -Boc-Lys(N$^\epsilon$-2ClZ) and N$^\alpha$-Boc-Glu($\gamma$-Bzl), respectively, in the coupling scheme to give Ac-Nle-Asp($\beta$-Bzl)-His (N$^{im}$-Tos)-D-Phe-Arg(N$^g$-Tos)-Trp(N$^i$-For)-Dab(N$^\gamma$ -Z)-p-MBHA resin. Upon cleaving and processing the peptide resin as reported for Example V, the peptide was obtained as a white powder in 36% yield.

EXAMPLE VIII

Ac-[Nle$^4$, Asp$^5$, D-Phe$^7$, Dpr$^{10}$]-alpha-MSH$_{4-10}$NH$_2$

The protected peptide resin to the title compound was synthesized as in Example V with the exception that N$^\alpha$-Boc-Dpr(N$^\beta$-Z) was used instead of N$^\alpha$-Boc-Lys-(N$^\epsilon$-2-ClZ) and N$^\alpha$-Boc-Asp($\beta$-Bzl) for N$^\alpha$-Boc-Glu($\gamma$-Bzl) in the coupling scheme to give Ac-Nle-Asp($\beta$-Bzl)-His-(N$^{im}$-Tos)-D-Phe-Arg(N$^g$-Tos)-Trp(N$^i$-For)-Dpr(N$^\beta$-Z)-p-MBHA resin. The peptide was cleaved from the resin, and processed as Example V to give the desired peptide as a white powder: yield 36%.

EXAMPLE IX

Ac-[Nle$^4$, Lys$^{10}$]-alpha-MSH$_{4-10}$NH$_2$

The protected peptide resin to the title compound was synthesized as reported in Example V, with the exception that N$^\alpha$-Boc-Phe was used instead of N$^\alpha$ -Boc-D-Phe in the coupling scheme to give Ac-Nle-Glu ($\gamma$-Bzl)-His(N$^{im}$-Tos)-Phe-Arg(N$^g$-Tos)-Trp(N$^i$-For)-Lys-(N$^\epsilon$-2-ClZ)-p-MBHA resin. The peptide was cleaved and processed to give the title compound with 40% yield.

EXAMPLE X

Ac-[Nle$^4$, Asp$^5$, Lys$^{10}$]-alpha-MSH$_{4-10}$NH$_2$

The protected peptide resin to the title compound was synthesized as reported in Example V, with the exception that N$^\alpha$-Boc-Phe was used instead of N$^\alpha$ -Boc-D-Phe and N$^\alpha$-Boc-Asp($\beta$-Bzl) was replaced N$^\alpha$ -Boc-Glu($\gamma$-Bzl) in the coupling scheme to give Ac-Nle-Asp($\beta$-Bzl)-Phe-Arg(N$^g$-Tos)-Trp(N$^i$-For)-Lys(N$^\epsilon$ -2-ClZ)-p-MBHA resin. The peptide was cleaved from the resin, the protecting groups removed, and the title compound purified as previously reported to give the product as a white powder in 37% yield.

The biological potencies for the linear analogues made in accordance with the preceding examples are given in the following table in which "P" indicates prolonged action (" + " = yes, "-" = No) and "ND" indicates the sample was not run for the specific test.

11

| Analogue | Biological Potency | | Residual Activity | |
|---|---|---|---|---|
| | Frog | Lizard | Frog | Lizard |
| alpha-MSH | 1.0 | 1.0 | P(-) | P(-) |
| 1 | 6.0 | 8.0 | P(+) | P(-) |
| 2 | 9.0 | 8.0 | P(+) | P(-) |
| 3 | 0.8 | 8.0 | P(+) | P(+) |
| 4 | 1.0 | 10.0 | P(+) | P(+) |
| 5 | 0.1 | 8.0 | P(-) | P(-) |
| 6 | 0.2 | 8.0 | P(-) | P(-) |
| 7 | 0.7 | 8.0 | P(-) | P(-) |
| 8 | 0.6 | 8.0 | P(-) | P(-) |
| 9 | 0.9 | 10.0 | ND | P(-) |
| 10 | 0.9 | 10.0 | P(±) | P(±) |
| 11 | 0.9 | 50.0 | P(-) | P(-) |
| 12 | 0.2 | 8.0 | P(-) | P(-) |
| 13 | 0.001 | 0.08 | P(+) | P(-) |
| 14 | 0.004 | 0.08 | P(+) | P(-) |

The results obtained with the linear conformationally restricted alpha-MSH analogues led to investigations using a different kind of restricted analogue, conformationally restricted cyclic alpha-MSH analogues.

The solid-phase peptide synthesis of cyclic melanotropin peptide analogues were conducted by conventional solid-phase synthetic techniques. In summary, $N^\alpha$-tert-butyloxycarbonyl (Boc) protected amino acids and their derivatives were coupled to a p-methylbenzhydrylamine resin with a 3-fold excess of the Boc-protected amino acid derivative, a 2.4-fold excess of N-hydroxybenzotriazole (HOBt) of 1 mmol/ml solution in DMF (except in case of His), and a 2.4-fold excess of 1 mmol/mL solution of dicyclohexylcarbodiimide (DCC) in DMF. The coupling reaction was carried out in dichloromethane for a 1 to 3 hour period, which were monitored by ninhydrin and/or chloranil tests and repeated as necessary. Reactive side chains of amino acids were protected as follows: Lys 2,4-dichlorobenzyloxycarbonyl; Orn, Dab, and Dpr benzyloxycarbonyl; Trp, formyl; Arg, tosyl; His, tosyl; Glu and Asp, Benzyl ester. Cleavage of the $N^\alpha$-Boc protecting group was performed by treatment with 48% trifluoroacetic acid containing 2% anisole in dichloromethane for 5 and 20 min each.

A cycle for the incorporation of each amino acid residue into the growing peptide chain consists of the following: 1) washing with $CH_2Cl_2$ (4 x 30 ml, 1 min/wash), 2) Boc protection was removed at each step by two treatments with 48% TFA in $CH_2Cl_2$ containing 2% anisole for 5 and 20 min each; 3) washing with $CH_2Cl_2$ (2 x 30 ml); 4) neutralizing with 10% diisopropylethylamine in $CH_2Cl_2$ (2 x 30 ml, 3 min/wash), 5) washing with $CH_2Cl_2$ (3 x 30 ml, 2 min/wash), 6) adding the Boc-protected amino acid derivative in 20 ml $CH_2Cl_2$ (except in the cases of Trp, Arg, and His when DMF was substituted for $CH_2Cl_2$ because of the solubility problem), followed by HOBt, followed by DCC and shaking for 1-3 h; 7) washing with $CH_2Cl_2$ (3 x 30 ml, 2 min/wash); and 8) washing with 100% EtOH (3 x 30 ml, 2 min/wash). Completion of coupling was monitored, and after coupling the last amino acid, the $N^\alpha$-Boc protecting group was removed, the amino group neutralized, and acetylated with a 10-fold excess of N-acetylimidazole in $CH_2Cl_2$ or using 1:1 mixture of acetic anhydride:pyridine in $CH_2Cl_2$ (2-fold excess for 1 h).

Peptides were deprotected and removed from the resin with anhydrous liquid HF (10 ml/1 g of resin) containing 10% anisole and 8% 1,2-dithioethane at 0°C for 45 min. After evaporation of the volatile materials in vacuo, the free peptides were washed with diethylether or ethyl acetate (3 x 30 ml) and then extracted with 30% aqueous solution of acetic acid (3 x 30 ml), and distilled water (3 x 30 ml). The combined aqueous extract was lyophilized to give a white powder of the crude peptide. Each peptide was purified by column chromatography on cation-exchange carboxymethyl cellulose (CMC) resin, using discontinuous gradient of ammonium acetate buffer as follows: 250 ml: of 0.01M $NH_4OAc$(pH 4.5), 250 ml of 0.01 M $NH_4OAc$(pH 6.8), 250 ml of 0.1 M $NH_4OAc$(pH 6.8), and 250 ml of 0.2 M $NH_4OAc$ (pH 6.8). The major peak (280 nm detection) eluted during the last part of 0.01 M $NH_4OAc$ (pH 6.8) and the first half of the 0.1 M $NH_4OAc$ (pH 6.8) buffer was lyophilized to give a purified peptide as a white powder.

The structure of the cyclic alpha-melanotropin analogues which comprise a portion of the present invention are set forth in the following table.

12

## Structure of Cyclic Alpha-Melanotropin Analogues

### Primary Sequence

| No. | 1 | 2 | 3 | 4 | 5 | 6 | 7 | 8 | 9 | 10 | 11 | 12 | 13 |

alpha-MSH: Ac-Ser-Tyr-Ser-Met-Glu-His-Phe-Arg-Trp-Gly-Lys-Pro-Val-NH$_2$

15: Ac-Nle-Glu-His-D-Phe-Arg-Trp-Gly-Lys-Pro-Val-NH$_2$

16: Ac-Nle-Glu-His-D-Phe-Arg-Trp-Lys-NH$_2$

17: Ac-Nle-Asp-His-D-Phe-Arg-Trp-Lys-NH$_2$

18: Ac-Nle-Asp-His-D-Phe-Arg-Trp-Orn-NH$_2$

19: Ac-Nle-Asp-His-D-Phe-Arg-Trp-Dab-NH$_2$

20: Ac-Nle-Asp-His-D-Phe-Arg-Trp-Dpr-NH$_2$

### Analogue

alpha-MSH

15: Ac-[Nle$^4$, Glu$^5$, D-Phe$^7$, Lys$^{10}$, Gly$^{11}$]-alpha-MSH$_{4-13}$NH$_2$

16: Ac-[Nle$^4$, Glu$^5$, D-Phe$^7$, Lys$^{10}$]-alpha-MSH$_{4-10}$NH$_2$

17: Ac-[Nle$^4$, Asp$^5$, D-Phe$^7$, Lys$^{10}$]-alpha-MSH$_{4-10}$NH$_2$

18: Ac-[Nle$^4$, Asp$^5$, D-Phe$^7$, Orn$^{10}$]-alpha-MSH$_{4-10}$NH$_2$

19: Ac-[Nle$^4$, Asp$^5$, D-Phe$^7$, Dab$^{10}$]-alpha-MSH$_{4-10}$NH$_2$

20: Ac-[Nle$^4$, Asp$^5$, D-Phe$^7$, Dpr$^{10}$]-alpha-MSH$_{4-10}$NH$_2$

The peptide analogues according to the present invention are prepared according to the following examples:

13

EXAMPLE XI

$$\text{Ac-[Nle}^4, \text{ Glu}^5, \text{ D-Phe}^7, \text{ Lys}^{10}, \text{ Gly}^{11}\text{]-Alpha-MSH}_{4-13}\text{NH}_2$$

Starting with 1.0 g of $N^\alpha$-Boc-Val-p-MBHA resin (0.7 mmol of $N^\alpha$-Boc-Val), the protected peptide resin for the title compound was prepared after stepwise coupling of the following $N^\alpha$-Boc-protected amino acids (in order of addition): $N^\alpha$-Boc-Pro; $N^\alpha$-Boc-Gly; $N^\alpha$-Boc-Lys($N^\epsilon$-2,4-ClZ); $N^\alpha$-Boc-Trp($N^i$-For); $N^\alpha$-Boc-Arg-($N^g$-Tos); $N^\alpha$-Boc-D-Phe; $N^\alpha$-Boc-His($N^{im}$-Tos); $N^\alpha$-Boc-Glu($\gamma$-Bzl); $N^\alpha$-Boc-Nle. After coupling the last amino acid, the $N^\alpha$-Boc protecting group was removed, the amino group neutralized, and acetylated with either 10-fold excess of N-acetylimidazole in dichloromethane (6-8 h) or with 2-fold excess of 1:1 mixture of acetic anhydride:pyridine in dichloromethane (1-2 h), and the resulting protected peptide resin Ac-Nle-Glu($\gamma$-Bzl)-His($N^{im}$-Tos)-D-Phe-Arg($N^g$-Tos)-Trp-($N^i$-For)-Lys($N^\epsilon$-2,4-ClZ)-Gly-Pro-Val-p-MBHA. A 1.0 g (0.6 mmol) portion of the protected peptide resin was treated with 10 ml anhydrous HF in the presence of 1 ml anisole and 0.8 ml 1,2-dithioethane for 45 min at 0°C. After the HF, anisole, and 1,2-dithioethane were evaporated in vacuo, the dried product mixture was washed with three 30 ml portions of diethylether, and the peptide was extracted with three 30 ml portions of 30% acetic acid. Then, upon lyophilization of the aqueous extract of the peptide, a 325 mg of crude Ac-Nle-Glu-His-D-Phe-Arg-Trp-Lys-Gly-Pro-Val-NH$_2$ peptide,as a white powder, was obtained. A 150 mg of crude Ac-[Nle$^4$, D-Phe$^7$, Lys$^{10}$, Gly$^{11}$]-alpha-MSH$_{4-13}$NH$_2$ was subjected to the purification scheme which included dissolving the crude peptide in 2-4 ml of 0.01 M NH$_4$OAc, pH 4.5, and chromatographed on carboxymethylcellulose column (2.0 x 25.0 cm) with a discontinuous gradient (250 ml each) of 0.01 (pH 4.5), 0.01, and 0.2 M NH$_4$OAc (pH 6.8). The major peak detected at 280 nm was eluted during the first half of the 0.1 M NH$_4$OAc (pH 6.8) buffer and was lyophilized to give 104 mg of a white powder. The CMC pure Ac-[Nle$^4$, D-Phe$^7$, Lys$^{10}$, Gly$^{11}$]-alpha-MSH$_{4-13}$NH$_2$ was further purified by HPLC, using 0.1% trifluoroactic acid buffer and acetonitrile as organic modifier on Vydac 218TP15-16 C$_{18}$RP (25 cm x 25 mm) semipreparative column. A 100 mg of the peptide was HPLC purified to give 74 mg pure Ac-[Nle$^4$, D-Phe$^7$, Lys$^{10}$,Gly$^{11}$]-alpha-MSH$_{4-13}$NH$_2$ peptide. A 40 mg sample of pure Ac-[Nle$^4$, D-Phe$^7$, Lys$^{10}$, Gly$^{11}$]-alpha-MSH$_{4-13}$ was dissolved in 1 ml of 5% HCl aqueous solution and chromatographed on diethylaminoethylcellulose (of hydrochloric acid form) column (1.0 x 15.0 cm) with 100 ml of 5% HCl, aqueous solution and the eluted peak monitored at 280 nm. Lyophilization of the collected peptide peak gave 35 mg of the Ac-[Nle, D-Phe$^7$, Lys$^{10}$, Gly$^{11}$]-alpha-MSH$_{4-13}$NH$_2$ x HCl salt. The peptide salt was dissolved in 3 ml of dry DMF and secondary amine free DMF (distilled from ninhydrin under reduced pressure). To the peptide solution in DMF was added anhydrous K$_2$HPO$_4$, the reaction mixture was cooled in an ice-salt bath to 0°C and 17 $\mu$l of diphenylphosphorylazide was added and the reaction mixture stirred at 0°C and then the whole reaction flask transferred to the cold room at 12°C. The reaction mixture was stirred overnight at 12°C and the completion of the reaction was monitored by HPLC (Vydac column, 25.0 cm x 4.6 mm with 0.1% trifluoroacetic acid/CH$_3$CN). Also, the ninhydrin test was used to detect the completion of the cyclization. The Ac-[Nle$^4$, Glu$^5$, D-Phe$^7$, Lys$^{10}$, Gly$^{11}$]-alpha-MSH$_{4-13}$NH$_2$ was purified, after quenching the reaction with 10% aqueous HOAc solution, by desalting on P$_4$ polyacryl amide column (80.0 cm x 1.0 cm) using 30% HOAc and purified by semipreparative HPLC to give 16 mg of cyclic peptide

$$\text{Ac-[Nle}^4, \text{ Glu}^5, \text{ D-Phe}^7, \text{ Lys}^{10}, \text{Gly}^{11}\text{]-alpha-MSH}_{4-13}\text{NH}_2.$$

EXAMPLE XII

$$\text{Ac-[Nle}^4\text{, Glu}^5\text{,D-Phe}^7\text{, Lys}^{10}\text{]-alpha-MSH}_{4-10}\text{NH}_2$$

The title compound was prepared starting with 2.0 g of $N^\alpha$-Boc-Lys($N^\epsilon$-2,4-Cl$_2$Z)-pMBHA resin ( 1.0 mmol/g of $N^\alpha$-Boc-Lys($N^\epsilon$-2,4,Cl$_2$Z), the protected peptide resin to the title compound was prepared after step-wise coupling of the following $N^\alpha$-Boc-protected amino acids (in order of addition): $N^\alpha$-Boc-Trp($N^i$-For); $N^\alpha$ -Boc-Arg($N^g$-Tos); $N^\alpha$-Boc-D-Phe; $N^\alpha$-Boc-His($N^{im}$-Tos). The resulting protected peptide resin, $N^\alpha$-Boc-His($N^{im}$-Tos)-D--Phe-Arg($N^g$-Tos)-Trp($N^i$-For)-Lys($N^\epsilon$-2,4-Cl$_2$Z)-p-MBHA resin was split into two halves. A 1.4 g ( 0.5 mmol) portion of the protected pentapeptide-resin was converted to the protected title peptide resin after coupling $N^\alpha$-Boc-Glu($\gamma$-Bzl) and $N^\alpha$-Boc-Nle. After coupling the last amino acid, the $N^\alpha$-Boc protecting group was removed, the amino group neutralized, and acetylated as reported in Example XI to give the protected peptide resin Ac-Nle-Glu($\gamma$-Bzl)-His($N^{im}$-Tos) -D-Phe-Arg($N^g$-Tos)-Trp($N^i$-For)-Lys($N^\epsilon$-2,4-Cl$_2$Z)-p-MBHA resin. A 1.0 g of the protected peptide resin was subjected to liquid HF cleavage and the peptide processed as in Example XI to give 356 mg of the crude Ac-[Nle$^4$, D-Phe$^7$, Lys$^{10}$]-alpha-MSH$_{4-10}$NH$_2$ peptide as a white powder. A 100.0 mg of the crude peptide was subjected to the purification scheme as outlined in Example XI to give 65 mg of HPLC pure Ac-[Nle$^4$, D-Phe$^7$, Lys$^{10}$]-alpha-MSH$_{4-10}$NH$_2$ peptide. 40 mg of this pure peptide was cyclized by the same approach as in Example XI to give 13 mg of HPLC pure

$$\text{Ac-[Nle}^4\text{, Glu}^5\text{, D-Phe}^7\text{, Lys}^{10}\text{]-alpha-MSH}_{4-10}\text{NH}_2\text{.}$$

EXAMPLE XIII

$$\text{Ac-[Nle}^4\text{, Asp}^5\text{ D-Phe, Lys}^{10}\text{]-alpha-MSH}_{4-10}\text{NH}_4$$

From 1.4 g ( 0.5 mmol) of Boc-His($N^{im}$-Tos)-D-Phe-Arg($N^g$-Tos) - Trp($N^i$-For)-Lys($N^\epsilon$-2,4-Cl$_2$Z)-p-MBHA resin the protected peptide resin of the title compound was prepared by stepwise coupling of $N^\alpha$-Boc-Asp($\beta$-Bzl) and $N^\alpha$-Boc-Nle. Each coupling reaction was achieved by following the same coupling scheme reported under the general solid-phase peptide methodology. After coupling the last amino acid, the $N^\alpha$-Boc protecting group was removed, the amino group neutralized, and acetylated as reported in Example XI, to give the protected peptide resin Ac-Nle-Asp($\beta$-Bzl) -His($N^{im}$-Tos)-D-Phe-Arg($N^g$-Tos)-Trp($N^i$-For)-Lys($N^\epsilon$ -2,4-Cl$_2$Z)-p-MBHA resin. A 1.0 g sample of the vacuum dried peptide resin was cleaved and processed as in Example XI to give 370 mg of the crude Ac-[Nle$^4$, Asp$^5$, D-Phe$^7$, Lys$^{10}$]-alpha-MSH$_{4-10}$NH$_2$. A portion of the crude heptapeptide (110 mg) was purified by the same procedure used in Example XI to give 82 mg of white powder of the linear precursor to the title peptide. A 40.0 mg of the pure Ac-[Nle$^4$, Asp$^5$,D-Phe$^7$, Lys$^{10}$]-alpha-MSH$_{4-10}$NH$_2$ was subjected to cyclization to give after processing the HPLC purification, 12 mg of pure

$$\text{Ac-[Nle}^4\text{,Asp}^5\text{, D-Phe}^7\text{, Lys}^{10}\text{]-alpha-MSH}_{4-10}\text{NH}_2\text{.}$$

15

EXAMPLE XIV

$$\text{Ac-[Nle}^4, \overline{\text{Asp}^5, \underline{\text{D-Phe}}^7, \text{Orn}^{10}}]\text{-alpha-MSH}_{4-10}\text{NH}_2$$

A 1.0 g of p-MBHA resin (0.7 mmol/g) was loaded with $N^\alpha$-Boc-Orn($N\gamma$-Z) using the coupling scheme reported in the general solid-phase procedure. After 1 h coupling the reaction stopped, the resin washed, neutralized and the free amino group on the resin acetylated with 2-fold excess of 1:1 mixture of acetic anhydride:pyridine in dichloromethane for 1 h. Then the following amino acids were successfully coupled to the resin by stepwise coupling: $N^\alpha$-Boc-Trp($N^i$-For); $N^\alpha$-Boc-Arg($N^g$-Tos); $N^\alpha$-Boc-D-Phe; $N^\alpha$-Boc-His($N^{im}$-Tos); $N^\alpha$-Boc-Asp($\beta$ Bzl); $N^\alpha$-Boc-Nle. Each coupling reaction was achieved by following the same coupling scheme outlined under the general solid-phase peptide methodology. After coupling the last amino acid, the $N^\alpha$-Boc protecting group was removed, the N-terminal amino group neutralized and acetylated to give the protected peptide resin Ac-Nle-Asp($\beta$-Bzl)-His($N^{im}$-Tos)-D-Phe-Arg($N^g$-Tos)-Trp($N^i$-For)-Orn($N\gamma$-Z)-p-MBHA resin. A 1.0 g portion of the vacuum dried peptide resin was cleaved and processed as outlined under Example XI to give 332 mg of the crude Ac-[Nle$^4$, Asp$^5$, D-Phe$^7$, Orn$^{10}$]-alpha-MSH$_{4-10}$NH$_2$. A 105 mg of the crude heptapeptide was purified by the method used in Example XI to give 78 mg of white powder of the linear peptide. A 40.0 mg sample of the pure Ac-[Nle$^4$, Asp$^5$, D-Phe$^7$, Orn$^{10}$]-alpha-MSH$_{4-10}$NH$_2$ was exposed to the cyclization procedure used for Example XI, to give, after proper workup, a product yield of 15 mg of pure

$$\text{Ac-[Nle}^4, \overline{\text{Asp}^5, \underline{\text{D-Phe}}^7, \text{Orn}^{10}}]\text{-alpha-MSH}_{4-10}\text{NH}_2.$$

EXAMPLE XV

$$\text{Ac-[Nle}^4, \overline{\text{Asp}^5, \underline{\text{D-Phe}}^7, \text{Dab}^{10}}]\text{-alpha-MSH}_{4-10}\text{NH}_2$$

1.0 g of p-MBHA resin (0.7 mmol/g) was coupled with $N^\alpha$-Boc-Dab($N\gamma$-Z) using the coupling scheme reported in the general solid-phase procedure. After 1 h coupling the reaction was stopped, the resin washed, neutralized and the unreacted amino group on the resin acetylated with 1-fold excess of 1:1 mixture of acetic anhydride:pyridine in dichloromethane for 1 h. Then, the following amino acids were successively coupled to the resin by stepwise coupling: $N^\alpha$-Boc-Trp ($N^i$-For); $N^\alpha$-Boc-Arg($N^g$-Tos); $N^\alpha$-Boc-D-Phe; $N^\alpha$-Boc-His($N^{im}$-Tos); $N^\alpha$-Boc-Asp-($\beta$-Bzl); and $N^\alpha$-Boc-Nle. After coupling the last amino acid, the $N^\alpha$-Boc protecting group was removed, the N-terminal amino group neutralized, and acetylated as reported in Example XI, to give the protected peptide resin Ac-Nle-Asp($\beta$-Bzl)-His($N^{im}$-Tos)-D-Phe-Arg($N^g$-Tos)-Trp-($N^i$-For)-Dab($N\gamma$-Z)-p-MBHA resin. A 1.0 g of the vacuum dried peptide resin was cleaved and processed to give 318.2 mg of the crude Ac-[Nle$^4$, Asp$^5$, D-Phe$^7$, Dab$^{10}$]-alpha-MSH$_{4-10}$NH$_2$. A 100.0 mg of the crude heptapeptide was purified to give 78.2 mg of white powder of the linear peptide. A 45.0 mg of the pure Ac-[Nle$^4$, Asp$^5$, D-Phe$^7$, Dab$^{10}$]-alpha-MSH$_{4-10}$NH$_2$ was cyclized and purified as previously discussed to give 13.2 mg of pure

$$\text{Ac-[Nle}^4, \overline{\text{Asp}^5, \underline{\text{D-Phe}}^7, \text{Dab}^{10}}]\text{-alpha-MSH}_{4-10}\text{NH}_2.$$

EXAMPLE XVI

$$\text{Ac-[Nle}^4, \text{Asp}^5, \underline{\text{D}}\text{-Phe}^7, \text{Dpr}^{10}]\text{-alpha-MSH}_{4-10}\text{NH2}$$

A 1.0 g of p-MBHA resin (0.7 mmol/g) was coupled with $N^\alpha$-Boc-Dpr($N^\beta$-Z) using the coupling scheme reported in the general solid-phase procedure. After 1 h coupling the reaction stopped, the resin washed, neutralized and the unreacted amino group on the resin washed, neutralized and the unreacted amino group on the resin acetylated with 2-fold excess of 1:1 mixture of acetic anhydride:pyridine in dichloromethane for 1 h. Then the following amino acids were successively coupled to the resin by stepwise coupling: $N^\alpha$-Boc-Trp($N^i$-For); $N^\alpha$-Boc-Arg($N^g$-Tos); $N^\alpha$-Boc-$\underline{\text{D}}$-Phe; $N^\alpha$-Boc-His($N^{im}$-Tos); $N^\alpha$-Boc-Asp($\beta$-Bzl); $N^\alpha$-Boc-Nle. After coupling the last amino acid, the $N^\alpha$-Boc protecting group was removed, the N-terminal amino group neutralized, and acetylated as in Example XI to give the protected peptide resin Ac-Nle-Asp($\beta$-Bzl)-His($N^{im}$-Tos)-D-Phe-Arg($N^g$-Tos)-Trp($N^i$-For)-Dpr($\beta$-Z)-p-MBHA resin. A 1.0 g of the vacuum dried peptide resin was cleaved and processed as outlined under 1 to give 310.8 mg of the crude Ac-[Nle$^4$, Asp$^5$, $\underline{\text{D}}$-Phe$^7$, Dpr$^{10}$]-alpha-MSH$_{4-10}$NH$_2$. A 115.0 mg sample of the crude heptapeptide was purified according to the procedure of Example XI to give 82.5 mg of white powder of the linear peptide. A 38.3 mg sample of the pure Ac-[Nle$^4$, Asp$^5$, $\underline{\text{D}}$-Phe$^7$, Dpr$^{10}$]-alpha-MSH$_{4-10}$NH$_2$ was cyclized and purified as previously discussed to give 11.3 mg of pure

$$\text{Ac-[Nle}^4, \text{Asp}^5, \underline{\text{D}}\text{-Phe}^7, \text{Dpr}^{10}]\text{-alpha-MSH}_{4-10}\text{NH}_2.$$

The biological potencies of alpha-MSH, the linear, and the cyclic analogues were determined by their ability to stimulate melanosome dispersion in vitro in the frog and lizard bioassays. These in vitro assays provide clear cut dose response curves (between $2.5 \times 10^{-11}$ and $4 \times 10^{-10}$ M) and can detect minimal concentration of alpha-MSH of about $10^{-11}$ M. The assays are based upon the centrifugal dispersion of melanosomes (melanin granules) within the dendritic processes of integumental dermal melanophores leading to darkening of the skin. All the test solutions were prepared via serial dilutions from a stock solution ($10^{-4}$ M). The frogs (Rans pipiens) used in these evaluations were obtained from Kons Scientific, Germantown, WI, and the lizards (Anolis carolinensis) were from the Snake Farm, La Place, LA. Biological activities of the cyclic alpha-melanotropin analogues are reported in the following table:

| Analogue | Biological Potency | | Residual Activity | |
|---|---|---|---|---|
| | Frog | Lizard | Frog | Lizard |
| alpha-MSH | 1.0 | 1.0 | p(-) | p(-) |
| 15 | 1.0 | 6.0 | p(+) | p(+) |
| 16 | 0.5 | 9.0 | p(+) | p(+) |
| 17 | 0.5 | 90.0 | p(+) | p(+) |
| 18 | 1.0 | 20.0 | p(-) | p(-) |
| 19 | 1.0 | 5.0 | p(-) | p(-) |
| 20 | 0.01 | 5.0 | p(-) | p(-) |

Our previous patents (US-A-4,457,864 and 4,485,039), the disclosures of which are incorporated in toto by reference, indicated the importance of conformational restriction of the active site of alpha-MSH. Cyclization constraint of alpha-MSH at positions 4 and 10 using pseudoisosteric replacement of Met-4 and Gly-10 with Cys amino acids resulted in many fold enhancement in melanocyte dispersion activity of the synthesized analogue. The potency enhancement of the cyclic analogues compared to that of their linear ancestor was clearly dependent on the bioassay system used. Our major interest was to get analogues with high potency on lizard skin since the activity of the latter bioassay is linearly correlated to the activity in the mammalian systems. The major characteristics of

$$[\overbrace{Cys^4, \quad Cys^{10}}]-alpha-MSH$$

cyclic analogues were that they showed low activity in lizard skin bioassay and high potency in frog skin; also with 500-fold reduction in activity upon ring size reduction (22-membered ring) or increasing (24-membered ring) in comparison with the optimized ring size (23-membered ring). The conformational constraint of alpha-MSH by lactam linkage between amino acids in positions 5 and 10 resulted in a group of cyclic compounds which comprise a portion of the present invention. The ring size centered at 23-membered ring, compound 17 gave the highest biological potency in lizard skin with not much change in potency in frog skin assay. The enhancement in potency between the cyclic and its linear ancestor is in the order of ten times of magnitude. The potency of cyclic analogue 16 is not much affected in comparison to the linear but with one important change with the appearance of prolonged activity in the cyclic analogue. The C-terminal tripeptide, Gly-Pro-Val has no effect on the potency of these analogues as is revealed in compound 15. There is one important factor in these cyclic lactam peptides, which is the prolongation in activity of the cyclic in comparison to that found in the linear. The compounds of the present invention are superior to alpha-MSH in one or more of the following characteristics: potency as measured by the in vivo and in vitro frog and/or lizard assay; duration of in vivo effect in such assays; and/or resistance to degradation by blood serum enzymes.

The compounds useful in this invention may be administered transdermally, and are formulated in suitable compositions determined by the intended means of administration, according to methods and procedures well-known to those skilled in the art. For example, the compounds suitable for use in this invention may be formulated or compounded with various conventional bases into preparations such as creams, ointments, gels, lotions, or sprays depending upon the desired mode of application of the ingredients to the skin of an individual. In manufacturing these preparations, the composition may also be mixed with conventional thickening agents, emollients, surfactants, pigments, perfumes, preservatives, fillers, and emulsifiers, all of which are well known and conventionally used in the formulation of transdermal preparations. Typically, these nonactive ingredients will make up the greater part of the final preparation. Preferably, the compositions are manufactured to allow for slow-release or timed-release delivery.

The remarkable properties of compounds of the invention also render them useful as substitutes for alpha-MSH and [Nle$^4$]-alpha-MSH in existing diagnostic, therapeutic and basic research schemes. In the area of diagnostic procedures, it is apparent that compounds of the invention, especially those which have been radioiodinated or coupled with gamma radiation emitters, are exceptionally well suited for use in locating and/or differentially characterizing melanoma cells on the basis of association with melanotropin receptors in such cells. The serum stability of compounds of the invention makes them prime candidates in proposed selective drug delivery systems wherein target tissues are known to have high concentrations of melanotropin receptors. The relative high potency and prolonged activity of compounds of the invention in color change-associated phenomena is expected to be duplicated in the context of other biological effects previously noted for naturally occurring melanocyte stimulating hormone and its synthetic analogues.

**Claims**

1. A linear analogue of alpha-MSH which is an Ac-alpha-MSH$_{4-10}$NH$_2$ analogue of the general formula:
   Ac[Nle$^4$, Xxx$^5$, D-Phe$^7$, Yyy$^{10}$]alpha-MSH$_{4-10}$NH$_2$
   wherein X$_{xx}$ is Glu or Asp, and Y$_{yy}$ is Lys, Orn, Dab or Dpr.

2. A linear alpha-MSH analogue selected from the group consisting of:

```
Ac-Ser-Tyr-Ser-Nle-Glu-His-D-Phe-Arg-Trp-Lys-Gly-Pro-Val-NH₂
Ac-Ser-Tyr-Ser-Nle-Asp-His-D-Phe-Arg-Trp-Lys-Gly-Pro-Val-NH₂
        Ac-Nle-Glu-His-D-Phe-Arg-Trp-Lys-Gly-Pro-Val-NH₂
        Ac-Nle-Asp-His-D-Phe-Arg-Trp-Lys-Gly-Pro-Val-NH₂
                Ac-Nle-Asp-His-D-Phe-Arg-Trp-Gly-NH₂
                Ac-Nle-Glu-His-D-Phe-Arg-Trp-Lys-NH₂
                Ac-Nle-Asp-His-D-Phe-Arg-Trp-Lys-NH₂
                Ac-Nle-Glu-His-D-Phe-Arg-Trp-Orn-NH₂
                Ac-Nle-Asp-His-D-Phe-Arg-Trp-Orn-NH₂
                Ac-Nle-Glu-His-D-Phe-Arg-Trp-Dab-NH₂
                Ac-Nle-Asp-His-D-Phe-Arg-Trp-Dab-NH₂
                Ac-Nle-Asp-His-D-Phe-Arg-Trp-Dpr-NH₂
                 Ac-Nle-Glu-His-Phe-Arg-Trp-Lys-NH₂
                 Ac-Nle-Asp-His-Phe-Arg-Trp-Lys-NH₂
```

3. A cyclic alpha-MSH analogue selected from the group consisting of:

```
      ┌──────────────────────┐
Ac-Nle-Glu-His-D-Phe-Arg-Trp-Lys-Gly-Pro-Val-NH₂
      ┌─────────────────────┐
Ac-Nle-Glu-His-D-Phe-Arg-Trp-Lys-NH₂
      ┌─────────────────────┐
Ac-Nle-Asp-His-D-Phe-Arg-Trp-Lys-NH₂
      ┌─────────────────────┐
Ac-Nle-Asp-His-D-Phe-Arg-Trp-Orn-NH₂
      ┌─────────────────────┐
Ac-Nle-Asp-His-D-Phe-Arg-Trp-Dab-NH₂
      ┌─────────────────────┐
Ac-Nle-Asp-His-D-Phe-Arg-Trp-Dpr-NH₂
```

4. A pharmaceutical preparation comprising a compound according to any preceding claim and a pharmaceutically-acceptable carrier or diluent.

5. A cosmetic preparation comprising a compound according to any one of Claims 1-3 together with a cosmetically-acceptable carrier or diluent.

6. The use of a compound according to any one of Claims 1-3 for the manufacture of a medicament having melanotropic activity.

7. The use of a compound according to any one of Claims 1-3 for the manufacture of a drug delivery system.

8. The use of a compound according to any one of Claims 1-3 for the manufacture of a diagnostic agent.

**Patentansprüche**

1. Ein lineares Alpha-MSH-Analogon, das ein Ac-Alpha-MSH$_{4-10}$NH$_2$-Analogon ist mit der allgemeinen Formel:

    Ac[Nle$^4$, Xxx$^5$, D-Phe$^7$, Yyy$^{10}$]Alpha-MSH$_{4-10}$NH$_2$
    worin X$_{xx}$ Glu oder Asp und Y$_{yy}$ Lys, Orn, Dab oder Dpr ist.

2. Ein lineares Alpha-MSH-Analogon, ausgewählt aus der Gruppe bestehend aus:

    Ac-Ser-Tyr-Ser-Nle-Glu-His-<u>D</u>-Phe-Arg-Trp-Lys-Gly-Pro-Val-NH$_2$

    Ac-Ser-Tyr-Ser-Nle-Asp-His-<u>D</u>-Phe-Arg-Trp-Lys-Gly-Pro-Val-NH$_2$

    Ac-Nle-Glu-His-<u>D</u>-Phe-Arg-Trp-Lys-Gly-Pro-Val-NH$_2$

    Ac-Nle-Asp-His-<u>D</u>-Phe-Arg-Trp-Lys-Gly-Pro-Val-NH$_2$

    Ac-Nle-Asp-His-<u>D</u>-Phe-Arg-Trp-Gly-NH$_2$

    Ac-Nle-Glu-His-<u>D</u>-Phe-Arg-Trp-Lys-NH$_2$

    Ac-Nle-Asp-His-<u>D</u>-Phe-Arg-Trp-Lys-NH$_2$

    Ac-Nle-Glu-His-<u>D</u>-Phe-Arg-Trp-Orn-NH$_2$

    Ac-Nle-Asp-His-<u>D</u>-Phe-Arg-Trp-Orn-NH$_2$

    Ac-Nle-Glu-His-<u>D</u>-Phe-Arg-Trp-Dab-NH$_2$

    Ac-Nle-Asp-His-<u>D</u>-Phe-Arg-Trp-Dab-NH$_2$

    Ac-Nle-Asp-His-<u>D</u>-Phe-Arg-Trp-Dpr-NH$_2$

    Ac-Nle-Glu-His-Phe-Arg-Trp-Lys-NH$_2$

    Ac-Nle-Asp-His-Phe-Arg-Trp-Lys-NH$_2$

3. Ein zyklische Alpha-MSH-Analogon, ausgewählt aus der Gruppe bestehend aus:

    Ac-Nle-Glu-His-<u>D</u>-Phe-Arg-Trp-Lys-Gly-Pro-Val-NH$_2$
    Ac-Nle-Glu-His-<u>D</u>-Phe-Arg-Trp-Lys-NH$_2$
    Ac-Nle-Asp-His-<u>D</u>-Phe-Arg-Trp-Lys-NH$_2$
    Ac-Nle-Asp-His-<u>D</u>-Phe-Arg-Trp-Orn-NH$_2$
    Ac-Nle-Asp-His-<u>D</u>-Phe-Arg-Trp-Dab-NH$_2$
    Ac-Nle-Asp-His-<u>D</u>-Phe-Arg-Trp-Dpr-NH$_2$

4. Ein pharmazeutisches Präparat bestehend aus einer Verbindung nach einem der obigen Ansprüche und einen pharmazeutisch akzeptablen Träger oder Verdünnungsmittel.

5. Ein kosmetisches Präparat bestehend aus einer Verbindung nach einem der Ansprüche 1 bis 3 zusammen mit einem kosmetisch akzeptablen Träger oder Verdünnungsmittel.

6. Die Verwendung einer Verbindung nach einem der Ansprüche 1 bis 3 zur Herstellung eines Medikaments mit melanotroper Wirkung.

7. Die Verwendung einer Verbindung nach einem der Ansprüche 1 bis 3 zur Herstellung eines Arzneimittel-Liefersystems.

20

EP 0 292 291 B1

8. Die Verwendung einer Verbindung nach einem der Ansprüche 1 bis 3 zur Herstellung eines Diagnosemittels.

**Revendications**

1. Un analogue linéaire d'alpha-MSH qui est un analogue Ac-alpha-$MSH_{4-10}NH_2$ de formule générale:
   Ac[Nle$^4$, Xxx$^5$, D-Phe$^7$, Yyy$^{10}$]alpha-$MSH_{4-10}NH_2$
   dans laquelle $X_{xx}$ est Glu ou Asp, et $Y_{yy}$ est Lys, Orn, Dab ou Dpr.

2. Un analogue linéaire d'alpha-MSH choisi parmi le groupe comprenant:

```
Ac-Ser-Tyr-Ser-Nle-Glu-His-D-Phe-Arg-Trp-Lys-Gly-Pro-Val-NH₂
Ac-Ser-Tyr-Ser-Nle-Asp-His-D-Phe-Arg-Trp-Lys-Gly-Pro-Val-NH₂
        Ac-Nle-Glu-His-D-Phe-Arg-Trp-Lys-Gly-Pro-Val-NH₂
        Ac-Nle-Asp-His-D-Phe-Arg-Trp-Lys-Gly-Pro-Val-NH₂
            Ac-Nle-Asp-His-D-Phe-Arg-Trp-Gly-NH₂
            Ac-Nle-Glu-His-D-Phe-Arg-Trp-Lys-NH₂
            Ac-Nle-Asp-His-D-Phe-Arg-Trp-Lys-NH₂
            Ac-Nle-Glu-His-D-Phe-Arg-Trp-Orn-NH₂
            Ac-Nle-Asp-His-D-Phe-Arg-Trp-Orn-NH₂
            Ac-Nle-Glu-His-D-Phe-Arg-Trp-Dab-NH₂
            Ac-Nle-Asp-His-D-Phe-Arg-Trp-Dab-NH₂
            Ac-Nle-Asp-His-D-Phe-Arg-Trp-Dpr-NH₂
             Ac-Nle-Glu-His-Phe-Arg-Trp-Lys-NH₂
             Ac-Nle-Asp-His-Phe-Arg-Trp-Lys-NH₂
```

3. Un analogue cyclique d'alpha-MSH choisi parmi le groupe comprenant:

```
        Ac-Nle-Glu-His-D-Phe-Arg-Trp-Lys-Gly-Pro-Val-NH₂
              └───────────────────────────────┘

            Ac-Nle-Glu-His-D-Phe-Arg-Trp-Lys-NH₂
                  └─────────────────────────┘

            Ac-Nle-Asp-His-D-Phe-Arg-Trp-Lys-NH₂
                  └─────────────────────────┘

            Ac-Nle-Asp-His-D-Phe-Arg-Trp-Orn-NH₂
                  └─────────────────────────┘

            Ac-Nle-Asp-His-D-Phe-Arg-Trp-Dab-NH₂
                  └─────────────────────────┘

            Ac-Nle-Asp-His-D-Phe-Arg-Trp-Dpr-NH₂
                  └─────────────────────────┘
```

4. Une préparation pharmaceutique contenant un composé selon l'une quelconque des revendications précédentes et un support ou un diluant pharmaceutiquement acceptable.

21

**5.** Une préparation cosmétique contenant un composé selon l'une quelconque des revendications 1 à 3 et un support ou un diluant accepté par la cosmétologie.

**6.** L'usage d'un composé selon l'une quelconque des revendications 1 à 3 pour l'élaboration d'un médicament possédant une activité mélanotrope.

**7.** L'usage d'un composé selon l'une quelconque des revendications 1 à 3 pour l'élaboration d'un système de libération de substance médicinale.

**8.** L'usage d'un composé selon l'une quelconque des revendications 1 à 3 pour l'élaboration d'un agent de diagnostic.